Europäisches Patentamt

(19) European Patent Office

Office européen des brevets

(11) Publication number: **0 285 429**
**A2**

(12) # EUROPEAN PATENT APPLICATION

(21) Application number: 88302916.7

(51) Int. Cl.⁴: **C 12 N 5/00**

(22) Date of filing: 31.03.88

(30) Priority: 31.03.87 JP 79974/87

(43) Date of publication of application:
05.10.88 Bulletin 88/40

(84) Designated Contracting States: **ES GR**

(71) Applicant: **UBE INDUSTRIES, LTD.**
**12-32, Nishihonmachi 1-chome**
**Ube-shi, Yamaguchi-ken 755 (JP)**

(72) Inventor: **Mori, Kazuhiro John Igarashichiku**
**Godoshukusha 1-102 7492-62 Igarashininomachi**
**Niigata-shi Niigata-ken (JP)**

**Doi, Shoichi**
**31-6 Iwakuranakaoosagimachi Sakyo-ku**
**Kyoto (JP)**

**Tezuka, Hiroaki Rakusaitakenosatodanchi 14-202 2-1**
**Ooharanohigashitakenosatomachi Nishikyo-ku**
**Kyoto-shi Kyoto (JP)**

**Nishikori, Masaru**
**21 Ichijyojisatonomaemachi Sakyo-ku**
**Kyoto-shi Kyoto (JP)**

(74) Representative: **Ritter, Stephen David et al**
**Mathys & Squire 10 Fleet Street**
**London EC4Y 1AY (GB)**

(54) Interleukin 3 producing cells and process for preparing interleukin 3.

(57) By employing the agar culture method developed by the present inventors, a human IL-3 producing human leukemia T cell line from mononuclear leukocytes in the peripheral blood of adult T cell leukemia patients. Desired human IL-3 is produced by cultivating this established cell line and is subsequently isolated. This human IL-3 has a mast cell growth activity and is capable of inducing the growth and differentiation of hemo-poietic cells including multi-potent stem cells, as well as many other cells.

EP 0 285 429 A2

## Description

### HUMAN INTERLEUKIN 3 PRODUCING CELLS AND PROCESS FOR PREPARING HUMAN INTERLEUKIN 3

FIELD OF THE INVENTION:

The present invention relates to human cells capable of producing human interleukin 3 having mast cell growth activity, as well as a process for preparing human interleukin 3 having mast cell growth activity.

BACKGROUND OF THE INVENTION:

Stem cells of marrow cells play important physiological roles by differentiating into lymphocytes, granulocytes, monocytes, platelets and erythrocytes, all of which are indispensable to living organisms for the maintenance and activity of their life. For such differentiation of bone marrow stem cells, their growth is of prime importance and is said to involve a variety of factors, such as a colony stimulating factor and interleukin 3 (hereinafter abbreviated as IL-3). IL-3 is a particularly important factor that acts upon multipotent hemopoietic stem cells to promote and control the growth and differentiation of various hemocytes.

Mouse IL-3 is known to be produced either from mouse spleen cells by stimulation with lectin or on the culture supernatant of a tumor cell line such as WEHI-3 [J. Immunol., 129, 1377 (1982)]. The cDNA of the gene of mouse IL-3 has been successfully cloned [Nature, 307 (19) 233 (1984)]. It has also been reported that human IL-3 is produced by transformation of human normal T cells with a human adult T leukemia virus (HTLV) [Science, 223, 703 (1984)]. Success in the cDNA cloning of the gene of human IL-3 has recently been reported [Cell, 47, 3 (1986)].

Mouse and human IL-3 have been named "IL-3" because a factor that had been known to have a unique capability for acting on undifferentiated T cells to induce 20α-hydroxy steriod dehydrogeanse (20α-SDH) and to differentiate it into mature T cells has been found to be identical to a mast cell growth factor (MCGF). Later on, cell lines such as FDCP-1 and -2 and DA-1 have been established as cells that depend on IL-3 for their growth and these cell lines have come to be used in the measurement of IL-3 activity.

However, it has recently been found that 20α-SDH can also be induced by a granulocyte macrophage colony stimulating factor (GM-CSF) and that cell lines FDCP-1 - 2 as well as DA-1 which had been established as IL-3 dependent cells are not qualified as specific markers for IL-3 because they can also be grown with the aid of interleukin 2 (hereinafter abbreviated as IL-2) or GM-CSF [Cellular and Molecular Biology of Lymphokines, 443-448 (1984), Academic Press, Inc. and Immunology Today, 7 (4) 104 (1986)]. In other words, the applicability of the methods for determination of IL-3 activity using the markers that had been considered to have specificity for IL-3 has been negated except for MCGF.

The methyl cellulose method has been known as a technique for the determination of MCGF activity and as will be noted below, this method is seemingly capable of measuring the MCGF activity but in fact is unable to provide the correct value of MCGF activity since it also has a high specificity for a macrophage colony stimulating factor (M-CSF). Therefore, no method has been known that is capable of correct measurement of MCGF activity.

Interleukin 3, which is a factor that acts on multi-potent hemopoietic stem cells to promote and control the growth and differentiation of various hemocytes, displays a multi-CSF activity in that it has a variety of CSF activities. Proof has been made of this fact with respect to mouse IL-3 but not for human IL-3. No factors in human that fully possess the multi-CSF activity, or IL-3 activity, have yet been reported to date. For instance, human IL-3 the cDNA of gene of which was successfully cloned [Cell, 47, 3 (1986)] has not been proved to have the multi-CSF activity.

DISCLOSURE OF THE INVENTION:

On the basis of this state of the art, the present inventors conducted intensive studies and found that the mouse T cell line they established had the ability to produce mouse IL-3. The present invention has been accomplished on the basis of this finding. In other words, the present inventors successfully established a mouse T cell line having the ability to produce mouse IL-3; they also succeeded in producing mouse IL-3 from this cell line.

The cell line established by the present inventors can be cultivated for a prolonged period on any of the media that are commonly employed in cultivation of animal cells and which may be optionally supplemented with serum. The cell line can also be cultivated in a serum- and protein-free medium.

By employing the agar culture method developed by the present inventors, the present inventors conducted various studies on human cell lines having the ability to produce human IL-3. In these studies, the present inventors cultivated the peripheral leukocytes of patients suffering from adult T cell leukemia (ATL) who showed bone marrow images that were as severe as those which would occur in chronic myelocytic leukemia (CML) and who had also contacted granulocytosis, and successfully established a human leukemia T cell line capable of producing human IL-3 having MCGF activity.

The cell line capable of producing human IL-3 established by the present inventors can be cultivated for a prolonged period on any of the media that are commonly employed in cultivation of animal cells and which may be optionally supplemented with serum. The resulting human IL-3 can be recovered by employing any of the known isolation and purification techniques such as salting-out, dialysis and ultrafiltration. If desired, the recovered human IL-3 can be purified to higher degrees by combinations of appropriate techniques such as ion-exchange chromatography, affinity chromatography and electrophoresis.

The so obtained IL-3 having MCGF activity are novel factors that has not yet been reported in the literature. This human IL-3 can be produced not only by cultivating the human leukemia T cells that have been established by the present inventors but also by cultivating human cells capable of producing this factor.

BRIEF DESCRIPTION OF THE DRAWINGS:

Fig. 1 shows the results of marker analysis of STIL-3 $C_5$ by flow cytofluorometry;

Fig. 2 shows the results of marker analysis of STIL-3 $D_{10}$ by flow cytofluorometry;

Fig. 3 shows the IL-3 producing activity of an L8313 mouse spleen cell conditioned medium which also produces granulocytes;

Fig. 4 shows the IL-3 producing activity of an L8313 transplanted mouse serum;

Fig. 5 shows a profile of mast cell production by the agar culture method in a way dependent on the concentration of STIL-3-CM;

Fig. 6 shows a profile of mast cell production from marrow cells by the agar culture method;

Fig. 7 is a photo showing the results of a chromosomal analysis conducted on cell line HIL-3;

Fig. 8 is a set of photos showing mouse mast cell colonies and human metachoromatic cell colonies induced by the culture supernatant of HIL-3 (IL-3), in which (1) shows mouse mast cells stained with toluidine blue, (2) shows mouse mast cells stained by the May-Grunwald-Giemsa technique, (3) shows human metachromatic cells stained with toluidiene blue, and (4) shows human metachromatic mast cell colonies stained with toluidine blue; and

Fig. 9 is a set of photos showing cells that are maintained in a human marrow cell suspending culture medium supplemented with the culture supernatant of HIL-3, in which (1) shows a megakaryocyte, (2) myeloblasts, and (3) basophiles, myeloblasts, proerythroblasts, etc.

REFERENCE EXAMPLE:

1. The mouse T cells capable of producing mouse IL-3 and the process for preparing mouse IL-3 using these cells are described hereinafter.

(1) Establishing mouse T cell line having the ability to produce mouse IL-3

(a) Radiation induced leukemia mouse

The radiation induced leukemia mice supplied from the Central Radiology Research Laboratory (4-9-1, Anagawa, Chiba, Japan) were used.

Eight to ten week old, male $C_3H$ mice were injected subcutaneously with 1 mg of predonisolone and irradiated to a total dose of 300 Rad so as to induce leukemia in the animals. The principal symptom of the disease was verified by the increased appearance of mature granulocytes in peripheral blood. Peripheral blood was collected from the leukemia-infected mice under anesthesia with ether by venipuncture under the eye and cytometry was conducted by measuring the numbers of leukocytes, erythrocytes and platelets with a cytometer.

(b) Establishing mouse IL-3 producing cell line (STIL-3)

A direct method was employed to establish an IL-3 producing cell line from the radiation induced leukemia mice obtained by the procedures described in (a). The direct method is described below.

Spleen cells collected from the leukemia-infected mice obtained by the procedures described in (a) were suspended in a 10% fetal calf serum (hereinafter abbreviated as FCS) containing RPMI-1640 medium at a density of $1 \times 10^6$ cells/ml, inoculated in a culture flask whose base was as large as 25 cm$^2$, and cultivated at 37°C under 5% $CO_2$.

After 3 days, half the volume of the medium was replaced by an equal volume of a fresh medium (culture by 2-fold dilution). The same procedures were followed every 3 days to establish a serially cultivatable mouse T cell line STIL-3 (hereinafter referred to as STIL-3).

STIL-3 can also be established by an indirect method such as an interstitial cell dependent cultivation technique or a nude mouse mediated technique. These alternative methods are described below.

(i) Interstitial cell dependent cultivation technique

Marrow cells from normal isologous $C_3H$ mice were cultivated for 3 weeks and the interstitial cells adherent on the wall of the culture bottle were irradiated with X-rays for a total dose of 800 Rad so as to suppress their growth and to kill the residual normal hemopoietic cells. The medium was washed with a fresh medium and plated with $1 \times 10^6$ spleen cells of L8313 mice per ml.

Cultivation by 2-fold dilution conducted once for every 3 - 7 days produced in 3 - 20 weeks a suspension of adherent cells that were growing on the adherent interstitial cells. These growing cells were collected to established STIL-3.

(ii) Nude mouse mediated technique

Nude mice were injected subcutaneously with $1 \times 10^7$ spleen cells of L8313 mice. In about 2 weeks, a tumor developed at the site of injection. The tumor was shredded and a single cell suspension was formed by pipetting. When the suspension was cultivated at a density of $1 \times 10^6$ cells/ml, a growth of cells dependent on interstitial cells first occurred as in (i), from which appeared a suspension of cells independent from intenstitial cells. The suspended cells were collected to establish STIL-3.

The cell line STIL-3 was treated by a limiting dilution technique to obtain single-cell derived clones of three new cell lines, STIL-3 $A_8$, $C_5$ and $D_{10}$.

The clones of STIL-3 $D_{10}$ were cultivated with the concentration of FCS being lowered stepwise to 0.2%. The clones that were capable of growing at the FCS concentration of 0.2% were transferred into Ham F12 + RPMI-1640 (1:1) medium and serially cultivated to establish a cell line capable of growing in a totally serum- and protein-free medium. This cell line was designated as STIL-3 $D_{101}$.

DNA was extracted from $1 \times 10^8$ cells of clones STIL-3 $C_5$, cut with restriction enzymes and subjected to Southern blotting, in which reconstruction of the T cell gene was verified using a marker for T cell interleukin 2 (hereinafter abbreviated as IL-2) receptor gene. Control was the DNA extracted from human placental cells.

As a result, it was found that STIL-3 $C_5$ was a mature T cell line having transposed DNA.

(2) Properties of mouse T cell line (STIL-3) having the ability to produce mouse IL-3

(a) Media for maintaining and growing T cell line

Cell line STIL-3 can be maintained and grown with high efficiency in RPMI-1640 medium containing 2 - 5% FCS. Cloned STIL-3 $A_8$, $C_5$ and $D_{10}$ grow at an FCS concentration of 2%; STIL-3 $D_{101}$ grows in a Ham F 12 + RPMI-1640 (1:1) medium, namely, a serum- and protein-free medium.

(b) Conditions for cell maintenance and growth of T line

Cell line STIL-3 grows well generally at 36 - 38°C, preferably at 37°C, with the pH controlled at 6.5 - 7.5, preferably at 7.2. Cultivation in an incubator filled with 5% $CO_2$ and 95% air is advantageous.

(c) Cell growth ability

When $1 \times 10^5$ cells/ml of this cell line are cultivated under the conditions shown above, a cell density of at least $2 - 3 \times 10^5$ cells/ml can be attained in 30 hours.

(d) Serial cultivation

Cell line STIL-3 can be serially cultivated without limitation. As of today which is more than one year after the start of cultivation, 60 - 150 generations of cells have been cultivated.

(e) Storage in a frozen state

After being frozen down to -80°C under specified conditions with a Programme Freezer of Cryo-Med Corp., cell line STIL-3 can be stored for a prolonged period either in a freezer or in liquid nitrogen without experiencing any substantial decrease in the number of viable cells.

(f) Cell surface marker

Spleen cells were incubated at 4°C for 30 minutes together with a saturated concentration of anti-Thy 1.2 antibody (Health Science Lado., Tokyo, Japan) and washed twice with a Hanks' conditioned physiological saline solution containing 1 mg/ml of bovine serum albumin and 0.1% sodium azine.

Thereafter, the cells were resuspended, incubated at 4°C for 30 minutes with FITC-bound second antibody, and washed.

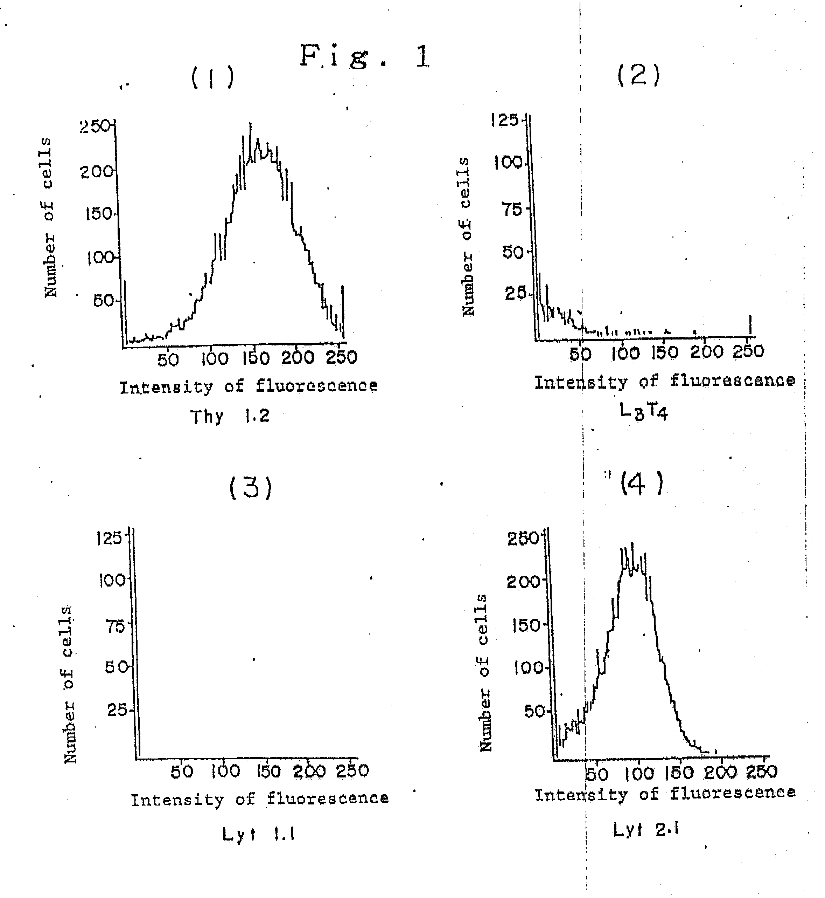

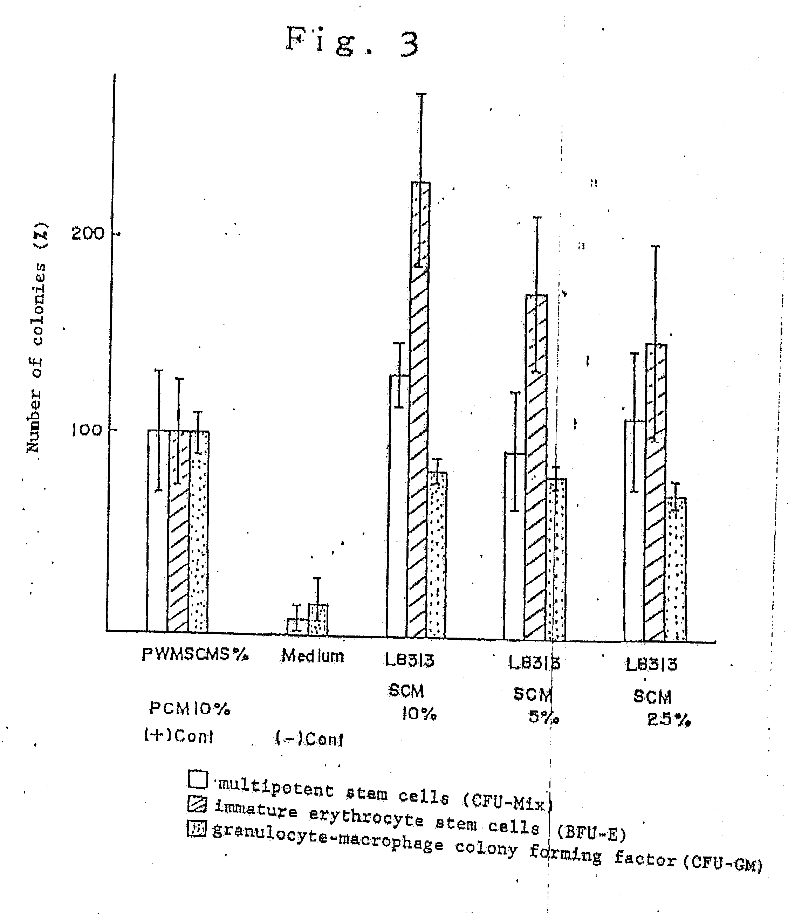

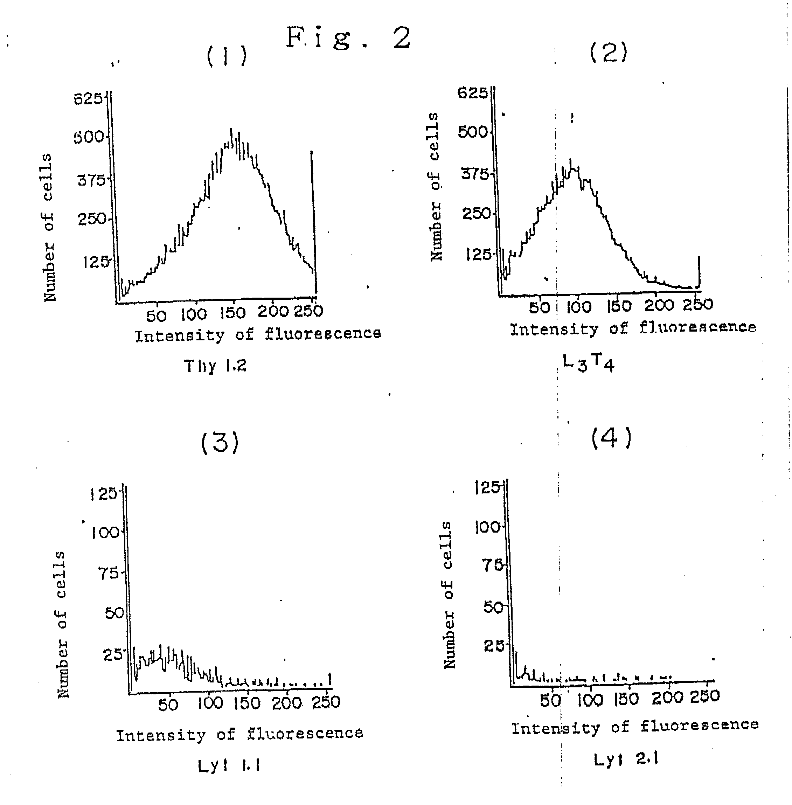

Thy 1.2 positive cells in the specimen were assayed with a flow cytofluorometric analyzer ($\lambda$, III; Ortho Diagnostic Systems, Waalwood, MA). The results obtained with STIL-3 $C_5$ and $D_{10}$ are shown in Figs. 1 and 2, respectively, from which one can see that STIL-3 $C_5$ is a suppressor T cell whereas STIL-3 $D_{10}$ is a helper T cell.

(3) Verification of IL-3 production

The MCGF activity of the established STIL-3 and the multi-potent stem cell (multi-CSF) activity based on the MCGF activity were verified by the following experiments so as to confirm the ability of STIL-3 to produce IL-3.

(a) MCGF activity of STIL-3 conditioned medium

An STIL-3 conditioned medium was prepared by cultivation of STIL-3. Mouse marrow cells ($1 \times 10^5$) were cultivated on an agar medium containing the so conditioned medium (20% v/v) and 25% horse serum. Colonies were recovered at days 7 and 14 and stained both by the May-Grunwald-Giemsa technique and with toluidine blue so as to obtain colony counts.

At day 7, a total of $67 \pm 3$ colonigies formed, the major proportions of which were mast cell colonies containing metachromatic coarse granules, which were accompanied by a minor proportion of macrophage or GM colonies. At day 14, the colony size increased and a total of $137 \pm 8$ colonies formed, all of which were mast cell colonies. The results are shown in Table 1.

## Table 1

### Growth of Mast Cells in Marrow Cells as Assayed on Semi-solid Agar Medium

| Days of culture | Number of mast cell colonies | Number of granulo-cyte colonies | Number of GM-CSF colonies | Number of macro-phage colonies | Plating efficiency (per $10^5$ cells) |
|---|---|---|---|---|---|
| 7 | 67 ± 3 | 0 | 3 ± 1 | 11 ± 2 | 67 |
| 14 | 137 ± 8 | 0 | 0 | 0 | 137 |

The above results show that the medium conditioned

The above results show that the medium conditioned with STIL-3 established by the present inventors had the ability to produce 100% pure mast cell colonies in a yield as high as 100 - 200 cells per $1 \times 10^6$ mouse marrow cells.

(b) Activity of transplanted L8313 mouse spleen cell conditioned medium and serum for producing multi-potent stem cells (hereinafter abbreviated as CFU-Mix), granulocyte-macrophage forming cells (hereinafter CFU-GM) and immature erythrocyte stem cells (hereinafter BFU-E)

The spleen cell conditioned medium (SCM) and serum used were prepared by the following procedures.

Spleen cells ($1 \times 10^7$) of radiation induced leukemia mice were injected into normal isologous mice and after 3 weeks, spleen cells were extracted from three of these mice. The extracted cells were cultivated in a 10% FCS containing α-medium at 37°C for 3 days in the presence of 5% $CO_2$ until a cell density of $2 \times 10^6$ cells/ml was reached. After the cultivation, the conditioned medium was spun and the supernatant was stored at -20°C.

Serum was collected at days 8 and 22 after the injection of normal isologous mice with $1 \times 10^7$ spleen cells from L8313 mice.

Serum from normal mice was used as a control, and a medium (PWMSCM) conditioned with spleen cells from normal C3H mice that had been stimulated with pokeweed mitogen were prepared by the methods described in Johnson, G. R. and Metcalf, D. (1977), Pro. Nat. Acad. Sci., USA, 74, 3879 and Hara, H. and Ogawa, M. (1978), Am. J. Hematology, 4, 23, and used as an index of multi-CSF.

Assay of colonies was conducted using normal mouse marrow cells ($1 \times 10^5$ cells/ml/petri dish) which were cultivated together with 0.8% methyl cellulose, 1% bovine serum albumin, 30% FCS, $5 \times 10^{-5}$ M mercaptoethanol and 2 units of erythropoietin (Connaught Labs, Ltd., Willowdale, Ontario, Canada). The basic media used were standard authentic samples and the test samples prepared above, namely, a medium conditioned with L8313 transplanted mouse spleen cells and an α-medium containing serum thereof.

CFU-Mix, CFU-GM and BFU-E counts were obtained at days 2, 9 and 14 and quantitative determination was made by the method of Hara and Ogawa.

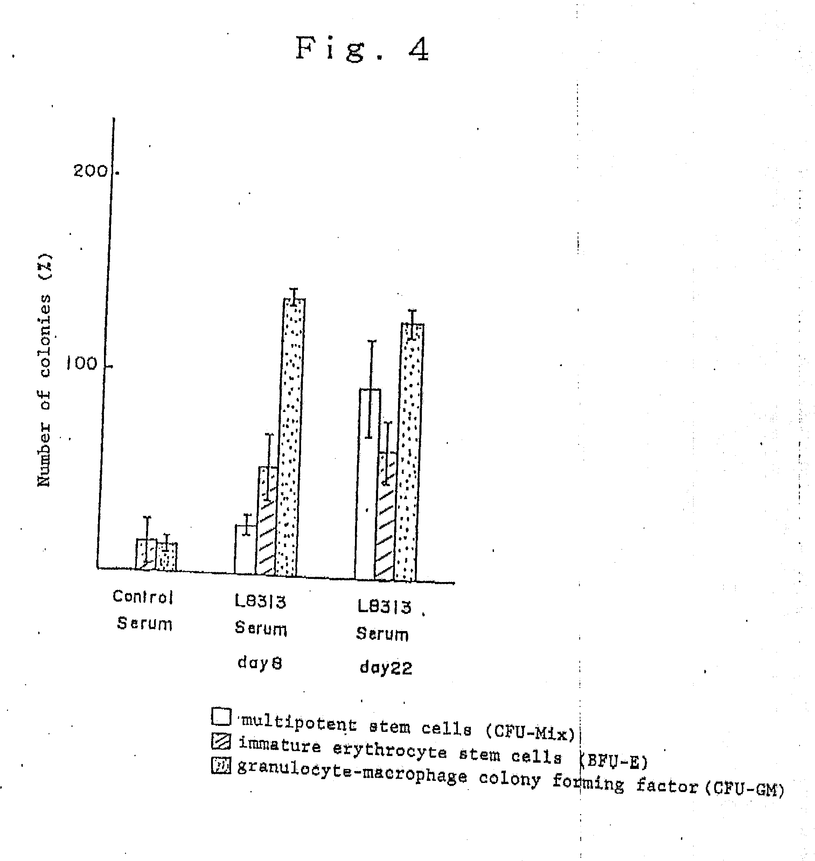

The results obtained with the concentration of the spleen cell conditioned medium being varied at 2.5%, 5% and 10% are shown in Fig. 3, from which one can see that the production of CFU-Mix, CFU-GM and BFU-E was dependent on the concentration of the spleen cell conditioned medium.

The results obtained with serum at days 8 and 22 after inoculation in L8313 mice are shown in Fig. 4, from which one can see the time-dependency of the production of CFU-Mix, CFU-GM and BFU-E.

(c) GM-CSF activity after treatment of L8313 mouse spleen cells with antibody and complement

Spleen cells extracted from three L8313 mice were treated with an anti-T cell antibody and a complement and cultivated for 3 days. The GM-CSF activity of the culture supernatant was measured at a final concentration of 10% (v/v). The control was normal mouse serum treated with a complement. The results are shown in Table 2.

## Table 2

### GM-CSF Activity after Treatment with Antibody and Complement

| Treatment | Number of colonies (per 1 x 10⁵ nucleated cells) |
|---|---|
| AWCM[a] | 98.7 ± 9.4 |
| NMS[b] + C' | 147.7 ± 17.0 (100%) |
| Thy 1.2 + C' | 10.7 ± 2.5 ( 7.6%) |
| Lyt 1.1 + C' | 145.0 ± 1.4 (102.0%) |
| Lyt 2.1 + C' | 118.0 ± 7.9 ( 83.3%) |
| Non-phagocytes[c] | 101.3 ± 4.5 ( 71.5%) |

a) Supernatant of 3-day culture of mouse ventral cells (GM-CSF)

b) Normal mouse serum

c) Non-Phagocytic cells

C': complement

As Table 2 shows, the spleen cells treated with a complement together with Thy 1.2 antibody as a cell surface marker had a lower GM-CSF activity and this indicates that in spleen cells, T cells are chiefly responsible for the production of GM-CSF.

(d) IL-3 activity of L8313 mouse spleen cells
  Production of IL-3 by T cells of L8313 mouse spleen cells was verified by the following experiment.
  To two cell lines, FDC-P2 and DA-1, the growth of which was dependent on IL-3, each of the culture supernatants used in (c) was added at a concentration of 10% (v/v) and the mixtures were cultivated for 24 hours in an RPMI-1640 medium containing 10% FCS.
  Thereafter, ³H-thymidine was added and cultivation was effected for an additional 6 hours. The cells were collected and the radioactivity of ³H-thymidine uptake was measured so as to examine the growth support activity, or IL-3 activity. The supernatant of the culture of IL-3 containing WEHI-3 was used as a positive control and each of FDC-P2 and DA-1 to which no supernatant was added was used as a negative control. In similar manners, the activity of STIL-3 C₅ and D₁₀ for IL-3 production was investigated. The results are shown in Table 3.

## Table 3

### IL-3 Activity Determined by Cell Growth Assay

| Sample | FDC-P2 | DA-1 (cpm) |
|---|---|---|
| Neg. cont. | 943 ± 89 | 846 ± 82 |
| WEHI-3 CM | 633815 ± 48568 | 49877 ± 2908 |
| L 8313 SCM | 256598 ± 43247 | 31420 ± 2006 |
| NMS + C' | 580780 ± 21780 | 34791 ± 1249 |
| Thy 1.2 + C' | 91866 ± 7352 | 5916 ± 523 |
| Lyt 1.1 + C' | 543810 ± 9112 | 52572 ± 2404 |
| Lyt 2.1 + C' | 469456 ± 42307 | 48083 ± 3125 |
| Non phagocytes | 626654 ± 16210 | 42706 ± 4399 |
| STIL-3 $C_5$ | 612260 ± 16114 | 55754 ± 6846 |
| STIL-3 $D_{10}$ | 479977 ± 29788 | 60402 ± 7669 |

(e) Multi-CSF Activity of STIL-3 $C_5$ and $D_{10}$

Each of STIL-3 $C_5$ and $D_{10}$ (2 x $10^5$ cells/ml) was cultivated for 3 days and the culture supernatants were assayed for IL-3 and multi-CSF activities at a final concentration of 10% (v/v).

A mouse abdominal wall conditioned medium and PWMSCM were used as controls. The results obtained in this experiment are shown in Table 4.

## Table 4

### Colony Forming Activity of STIL-3 Conditioned Medium

| Sample | CFU-GM | BFU-E | CFU-Mix |
|---|---|---|---|
| Control | 87.0 ± 3.1 | 6.3 ± 2.1 | 4.1 ± 0.3 |
| STIL-3 $C_5$ | 140.0 ± 22.0 | 5.0 ± 2.0 | 2.0 ± 0 |
| STIL-3 $D_{10}$ | 99.3 ± 4.2 | 6.0 ± 1.4 | 2.3 ± 0.5 |

The above data shows that the products of cell lines STIL-3 $C_5$ and $D_{10}$ have a multi-CSF activity.

(f) IL-2 producing activity of cell lines STIL-3 $C_5$ and $D_{10}$

IL-2 producing activity was examined for each of the cell lines STIL-3 $C_5$ and $D_{10}$ by the following procedures. Each of STIL-3 $C_5$ and $D_{10}$ (2 x $10^5$ cells/ml) was cultivated for 3 days and the culture supernatants were added to cell line CTIL-2 the growth of which was dependent on IL-2. The mixtures were cultivated for 24 hours in an RPMI-1640 medium containing 10% FCS. Thereafter, $^3$H-thymidine was added and cultivation was conducted for an additional 6 hours. The cells were collected and the radioactivity of $^3$H-thymidine uptake was measured so as to examine the IL-2 activity. Comparison was made against a standard sample (Neg. cont.), with 10% purified rat IL-2 being used as a positive control (Post. Cont.). The results are shown in Table 5.

## Table 5

### IL-2 Activity of STIL-3 Conditioned Medium

| Sample | CTIL-2 (cpm) |
|---|---|
| Neg. cont. | 1834 ± 136 |
| Post. cont. | 21399 ± 888 |
| STIL-3 $C_5$ | 822 ± 71 |
| STIL-3 $D_{10}$ | 1607 ± 95 |

The above data shows that none of STIL-3 $C_5$ and $D_{10}$ established by the present inventors had the ability to produce IL-2.

The above results established the fact that the factor produced by STIL-3 has both MCGF and multi-CSF activities and is the true mouse IL-3.

The activity of IL-3 produced by the cell line STIL-3 established in the present invention was measured by the MCGF activity assay technique that was newly developed by the present inventors and which is described hereinafter.

The methyl cellulose method has been known as a technique for the determination of MCGF activity and this method is seemingly capable of measuring the MCGF activity. In fact, however, this method has involved considerable difficulty in detecting the true MCGF activity since it also has a high specificity for a macrophage colony stimulating factor (hereinafter abbreviated as M-CSF). Under these circumstances, the present inventors conducted intensive studies and succeeded in establishing an agar culture method as a technique capable of providing the true value of MCGF activity. This has been verified by the comparative experiment to be described below.

Mouse marrow cells ($1 \times 10^5$) were cultivated on two IL-3 producing sources, one being a methyl cellulose medium supplemented with an STIL-3 conditioned medium (20% v/v) and the other being an agar medium also supplemented with STIL-3 conditioned medium (20% v/v). Colonies were recovered at days 7, 14 and 21 (at days 7 and 14 for the agar medium) and stained body by the May-Grunwald-Giemsa technique and with toluidine blue for obtaining colony counts.

As a standard for GM-CSF, CFU-GM was cultivated in a semi-solid $\alpha$-medium containing 0.8% methyl cellulose, 20% horse serum and a mouse abdominal wall conditioned medium at 37°C in the presence of 5% $CO_2$. A CFU-GM count was taken at day 7.

The results of MCGF activity assay conducted on the methyl cellulose media are shown in Table 6.

## Table 6

### Growth of Mast Cells in Bone Marrow as Assayed in Semi-solid Methyl Cellulose Medium

| Days of culture | Number of mast cell colonies | Number of granulo-cyte colonies | Number of GM-CSF colonies | Number of macro-phage colonies | Plating efficiency (per $10^5$ cells) |
|---|---|---|---|---|---|
| 7 | 0 | 73 ± 5 | 41 ± 8 | 219 ± 11 | 0 |
| 14 | 15 ± 2 | 0 | 18 ± 2 | 270 ± 17 | 15 |
| ·21 | 11 ± 2 | 0 | 0 | 198 ± 10 | 11 |

The data shows that the MCGF activity detected by the methyl cellulose method was too low to warrant the effectiveness of this method in providing correct measurements of MCGF activity.

In contrast, the agar culture method is capable of assaying the MCGF activity with 100% efficiency as shown in 3(a) and may well be considered as an effective technique for assaying the IL-3 activity.

The reliability of the agar culture method was also verified by assaying a WEHI-CM conditioned medium or a purified Ihle conditioned medium. The results of assay of mast cell colonies on the 7th day of cultivation of mouse marrow cells in an IL-3 containing agar medium are shown in Table 7.

### Table 7

### Activity for Mast Cell Colony Production by WEHI-CM and Purified Mouse IL-3

| IL-producing source | Number of mast cell colonies/ $10^5$ mouse marrow cells |
|---|---|
| WEHI-CM (30% v/v) | 180 ± 2 |
| Purified IL-3 (30 U/ml) | 160 ± 8 |

The results show that 80% of the colonies formed were mast cells, the remaining colonies being either granulocytes or macrophages.

An experiment was also conducted to stimulate mouse marrow cells with a granulocyte-macrophage colony stimulating factor (GM-CSF) and the type of each of the colonies formed was analyzed by the agar culture method. The assay was made by cultivating mouse marrow cells in a 0.3% agar medium. On the 7th day of cultivation, the colonies were stained by the May-Grunwald-Giemsa technique for taking colony counts. The results are shown in Table 8.

### Table 8

### Colony Type Analysis in Agar Medium for GM-CSF Stimulated Mouse Marrow Cells

| Number of colonies (per $10^5$ cells) | Colony type | | | | |
|---|---|---|---|---|---|
| | granulo-cyte | macro-phage | granulocyte macrophage | immatures | mast cells |
| 166 (100%) | 63 (38%) | 26 (16%) | 34 (20%) | 43 (26%) | ND (0%) |

ND: Not detected. Mast cells and eosinophilic cells were detected in small quantities but no colony formation was observed.

The above data shows that GM-CSF was incapable of producing mast cell colonies.

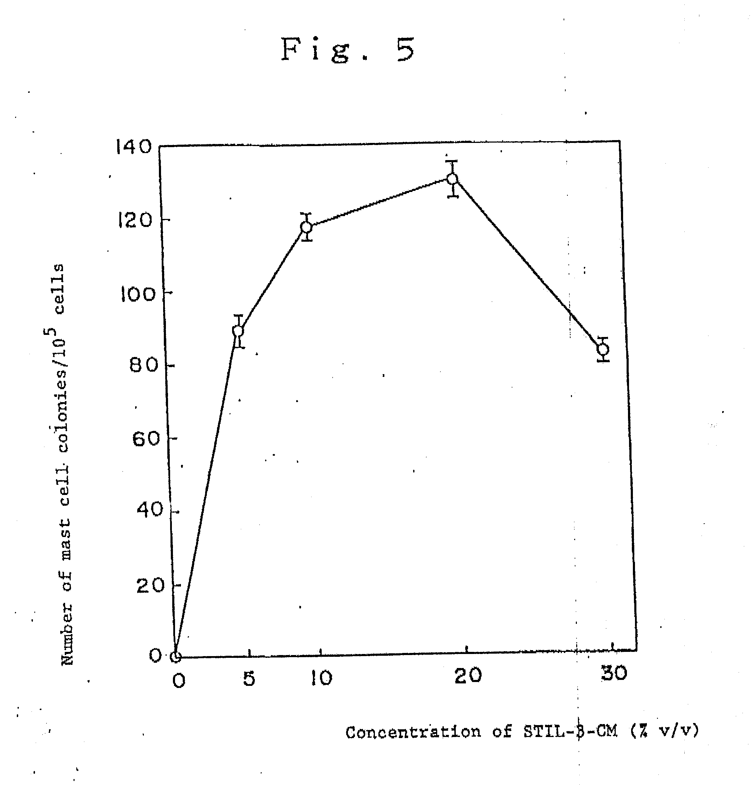

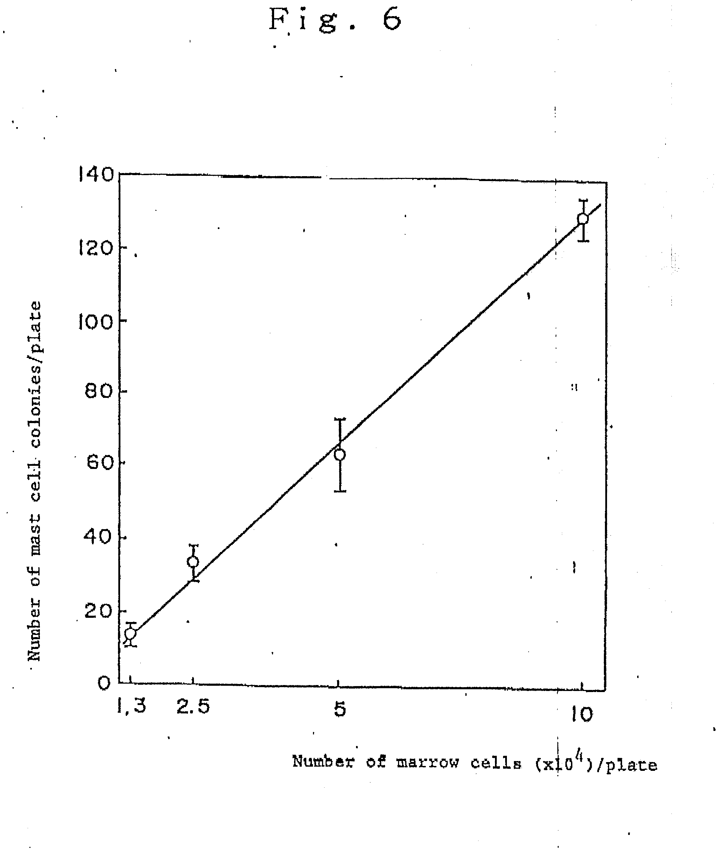

The number of mast cell colonies formed as a function of the concentration of STIL-3 conditioned medium (STIL-3CM) and the number of marrow cells was measured by the agar culture method and the results are shown in Figs. 5 and 6, from which one can see that IL-3 activity can be measured by the agar culture method in a quantitative and rapid way.

EXAMPLE

The human T cells capable of producing human IL-3 and the process for preparing human IL-3 using these cells are hereunder described in greater detail.

9

(1) Establishing human IL-3 producing cell line

(a) Isolating and establishing HIL-3 cell line

A peripheral blood sample was collected from a patient (Japanese woman aged 52) who was suffering from adult T cell leukemia complicated by granulocytosis and the mononuclear leukocytes were isolated. The isolated mononuclear leukocytes were cultivated in 10% FCS supplemented RPMI-1640 medium containing human IL-2 (2 U/ml) at 37°C in the presence of 5% $CO_2$. Cell growth started about one month later and has continued to date (the cultivation started on September 9, 1986) when sustained growth is still going on in the presence of 1 - 2 U/ml of IL-2. This shows that a serially cultivatable human T cell line (hereinafter abbreviated as HIL-3) has been established.

The cell line HIL-3 has been deposited with the Fermentation Research Institute, the Agency of Industrial Science &; Technology on February 24, 1988 under the Budapest Treaty under Accession No. FERM BP-1760.

(b) Establishing clones or subclones of HIL-3

Clones of HIL-3 can be obtained by standard techniques of selective cloning. Clones having high ability to produce IL-3 can be obtained by checking the quantity of IL-3 production when cloning of HIL-3 is effected by standard techniques.

Stated more specifically a total of $1 \times 10^6$ HIL-3 cells were diluted to 0 - 1 cells per well by a limiting dilution technique and subjected to repeated cultivation at 37°C in the presence of 5% $CO_2$ so as to select cell clones having great IL-3 producing ability.

(2) Properties of human T cell line HIL-3

(a) Media for maintaining and growing HIL-3

Cell line HIL-3 can be maintained and growth with high efficiency in RPMI-1640 medium containing 10% FCS and 1 - 2 U/ml of IL-2. This cell line can also be maintained in an IL-2 free medium.

(b) Conditions for cell maintenance and growth and the dependency of cell growth

Cell line HIL-3 grows well generally at 36 - 38°C, preferably at 37°C, with the pH controlled at 6.5 - 7.5, preferably at 7.2. Cultivation in an incubator filled with 5% $CO_2$ and 95% air is advantageous.

(c) Cell growth ability

When $2 \times 10^5$ HIL-3 cells/ml are cultivated under the conditions shown above, a cell density of at least $5 \times 10^5$ cells/ml can be attained in 3 days.

(d) Serial cultivation

Cell line HIL-3 can be serially cultivated without limitation. As of today which is more than one year after the start of cultivation, 63 generations of cells have been cultivated.

(e) Storage in a frozen state

After being frozen down -80°C under specified conditions with a Programme Freezer of Cryo-Med Corp., cell line HIL-3 can be stored for a prolonged period either in a freezer or in liquid nitrogen without experiencing any substantial decrease in the number of viable cells.

(f) Morphological properties

HIL-3 cells grow in the log phase forming colonies each consisting of several tens of cells. The cells are of a medium to large size and have a circular shape, sometimes, being irregularly shaped with pseudopodium-like protrusions.

Specimens stained by the May-Grunwald-Giemsa technique have basophilic cytoplasm and one or two circular nuclei.

(g) Properties of chromosome

The chromosomes of HIL-3 cells are of the human female type and the structural abnormality common to all karyotypes analyzed was inversion of the long arm of chromosome No. 14, namely, inv (14) (q11 q32). The chomomoes examined had the karyotype 46, XX, inv (14) (q11 q32).

(h) Verification by chromosome analysis

HIL-3 cells growing most actively in the presence of IL-2 were treated with Colcemid for 60 minutes, subjected to a hypotonic treatment with 0.075 M KCl for 20 minutes, and fixed with a Carnoy fixing fluid. The chromosomal specimens were spread on a glass slide by the air-drying method, photographed by the G-band method and analyzed.

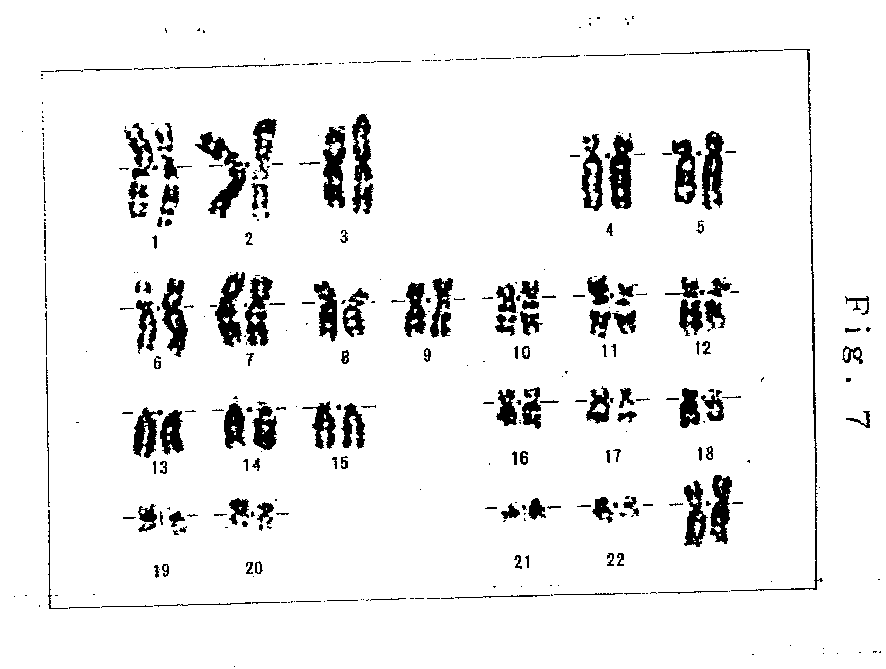

For the identified karyotype, 8 cells in the metaphase of division were analyzed. All cells had an inverted long chain in chromosome No. 14 [inv (14) (q11 q32)]. This marker chromosome had malformation in 6 cells and the remaining 2 cells had two additional chromosome malformations, one being an external chromosome X (+X) and the other being 15q + chromosome [der (15) t (15;?) (q25;?)]. The results of these analyses are shown in Fig. 7.

(i) Number of chromosomes
   46, XX, inv (14) (q11 q32)

(j) Cell surface marker
   All markers were stained by the indirect fluorescent antibody method and assayed for the percent positive cell either under a fluorescent microscope or with the naked eye.
   The results showed that cell line HIL-3 formed E-rosettes, were positive with respect to anti-human HLA-DR (HLA-DR), anti-T lymphocyte antibody (OKT 1, OKT 3), antihelper T cell antibody (OKT 4), anti-ED set receptor antibody (OKT 11) and anti-IL-2 receptor antibody (Tac), and were negative with respect to anti-suppressor T cell antibody (OKT 8), anti-B cell antibody (B1) and anti-TdT antibody. These results are summarized in Table 9.

## Table 9

### Cell Surface Marker of HIL-3

#### Phenotype

| | | |
|---|---|---|
| E-rosette | 84% | (+) |
| HLA-DR | 97% | (+) |
| OKT 1 | 95% | (+) |
| OKT 3 | 67% | (+) |
| OKT 4 | 79% | (+) |
| OKT 8 | 0% | (−) |
| OKT 11 | 96% | (+) |
| Tac | 95% | (+) |
| B1 | 0% | (−) |
| TdT | 0% | (−) |

The data shown in Table 9 reveals that cell line HIL-3 is a T cell line.

(3) IL-3 Producing Activity of HIL-3 Cell Line

(a) Measuring IL-3 activity with mouse marrow cells used as target cells
   Marrow cells derived from a mouse femoral bone were brought into single cells in an α-medium using a syringe needle (26G), and $5 \times 10^4$ such single cells per ml were added to an α-medium supplemented with 25% potato (HS), 0.3% agar and 0 - 40% of the culture supernatant of HIL-3. Thereafter, the medium was placed in 2-ml portions in Petri dishes 30 mm in dia. (Falcon) and subjected to cultivation for 7 - 14 days at 37°C in the presence of 5% $CO_2$ at 100% humidity. The colony counts obtained at days 7 and 14 are shown in Table 10 in terms of the mean ± SD of colonies from 3 petri dishes per $10^5$ mouse marrow cells.

## Table 10

### Cultivation of Mouse Marrow Cells in Semi-solid Agar Medium in the Presence of the Culture Supernatant of HIL-3

| Concentration of culture supernatant of HIL-3 (%) | day 7 | day 14 |
|---|---|---|
| 0 | 0 | 0 |
| 5 | 50 ± 3.6 | 11 ± 1.0 |
| 10 | 191 ± 15.9 | 100 ± 9.5 |
| 20 | 248 ± 12.2 | 181 ± 11.5 |
| 30 | 403 ± 39.8 | 255 ± 20.1 |
| 40 | 396 ± 40.7 | 272 ± 14.4 |

The data shows that the culture supernatant of HIL-3 cells induced differentiation of mouse marrow cells into mast cells in a yield of about 80% of all the colonies at day 7 and in a yield of 95% at day 14.

(b) Measurement of IL-3 activity with human marrow cells used as target cells

A normal human bone marrow was collected with a heparinized syringe and the monocnuclear leukocytes separated with a Ficoll-Hypaque were washed three times with an $\alpha$-medium. The marrow cells were prepared at a concentration of $5 \times 10^4$ cells/ml in an $\alpha$-medium supplemented with 25% HS, 0.3% agar and 5 - 30% of the culture supernatant of HIL-3, and cultivated in petri dishes (Falcon) 30 mm in diameter. The cultivation was continued for 7 - 14 days in an incubator at 37°C in the presence of 5% $CO_2$ with the humidity maintained at 100%. The colony counts obtained at days 7 and 14 are shown in Table 11 in terms of the mean ± SD of colonies from 4 petri dishes per $5 \times 10^4$ normal human marrow cells.

## Table 11

### Cultivation of Human Marrow Cells in Semi-solid Agar Medium in the Presence of the Culture Supernatant of HIL-3

| Concentration of culture supernatant of HIL-3 (%) | day 7 | day 14 |
|---|---|---|
| 5 | $10 \pm 2.0^a$ | NT |
| 10 | $16 \pm 1.2$ | NT |
| 20 | $22 \pm 1.0^a$ | $59 \pm 9.9^b$ |
| 30 | $30 \pm 1.0$ | NT |

a:  Half of the colonies observed were metachromatic cells stained with toluidine blue.

b:  About 80% of the colonies observed were mast cell colonies.

NT:  Not tested.

The above results show that the culture supernatant of HIL-3 cells had the ability to produce 100 - 400 colonies of 100% pure basophilic cells per $10^5$ human marrow cells.

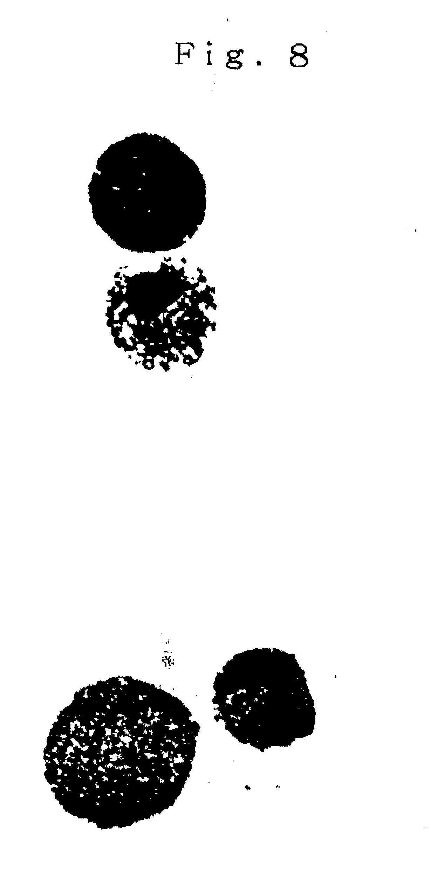

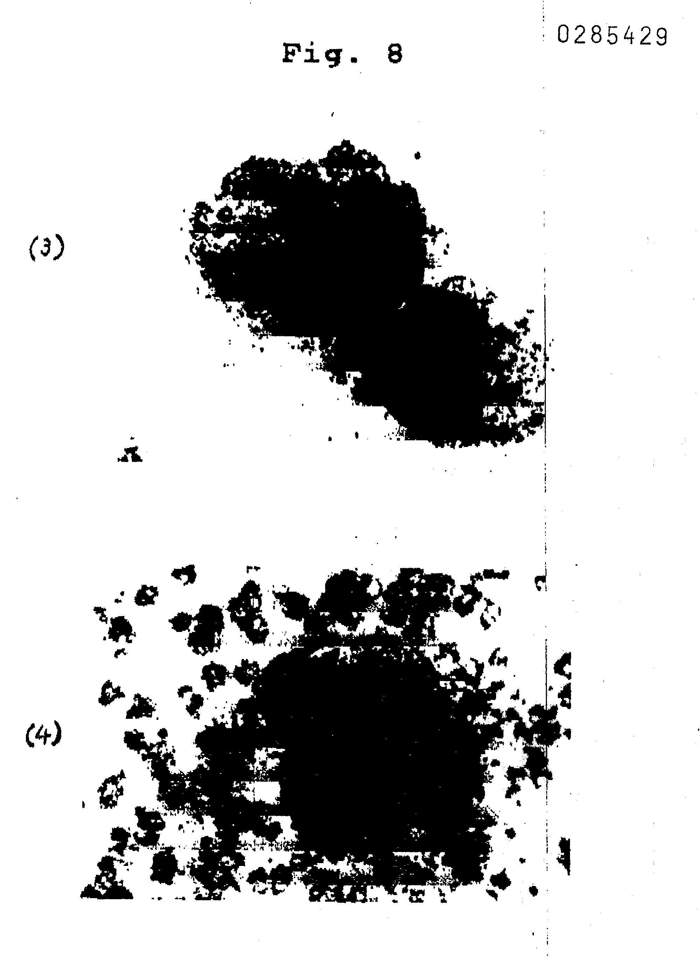

The metachromatic cells obtained would most probably be mast cells. Fig. 8 shows the mast cell colonies [(1) and (2)] and metachromatic cell colonies [(3) and (4)] produced by the culture supernatant of HIL-3.

Figs. 8(1) and (2) show the results of staining with toluidine blue and by the May-Grüwald-Giemsa technique, respectively, of specimens prepared from colony forming cells with cytospin 2 (Chandon), said cells having been produced by cultivation of mouse marrow cells in a medium containing the culture supernatant of HIL-3. Figs. 8(3) and (4) show the result of staining with toluidine blue of an air-dried and fixed medium on a glass slide as it contained colonies forming on the 14th day of cultivation of human marrow cells in a medium supplemented with the culture supernatant of HIL-3.

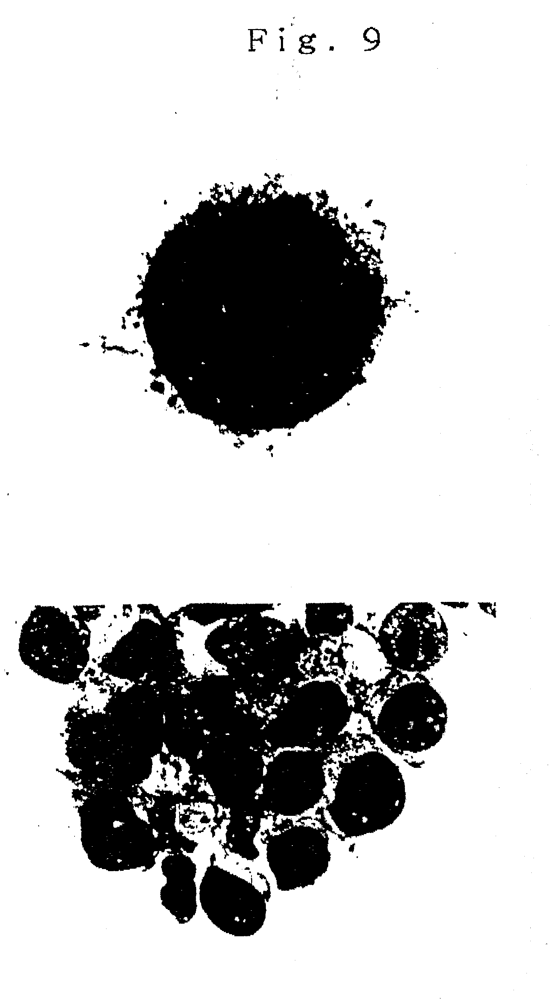

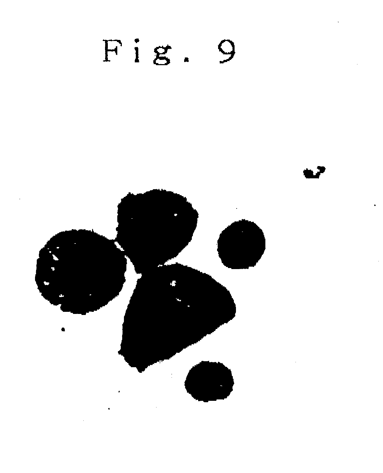

In the next place, $1 \times 10^5$/ml of human marrow cells were added to an α-medium containing 20% HS and 20% culture supernatant of HIL-3 and cultivated. The cultivation was continued at 37°C in the presence of 5% $CO_2$ at 100% humidity, with half of the liquid medium being replaced by a fresh one every 7 days. As a result, it was found to be possible to maintain megakaryocytes, myeloblasts, etc. at week 4 as Fig. 9 shows.

Fig. 9(1) shows one of the megakaryocytes obtained at week 4 of the cultivation; Fig. 9(2) shows the myeloblasts obtained at week 4 of the cultivation; and Fig. 9(3) shows the proerythroblasts, basophiles, myeloblasts, etc. obtained at week 4 of the cultivation.

(c) Cultivation of mouse marrow cells in a Methyl Cellulose system in the presence of the culture supernatant of HIL-3

Mouse marrow cells ($1 \times 10^5$ cells) were cultivated in the presence of 0.8% methyl cellulose, 30% FCS, 1% BSA, $1 \times 10^{-4}$ M mercaptoethanol and an the IL-2 free culture supernatant of HIL-3, and juvenile erythrocyte precursor cells (hereunder abbreviated as BFU-E) and multi-potent stem cells (hereunder abbreviated as CFU-Mix) were produced. The results are shown in Table 12 in terms of the mean ± SD of colonies from 3 petri dishes per $1 \times 10^5$ marrow cells.

## Table 12

### Cultivation of Mouse Marrow Cells in Methyl Cellulose System in the Presence of Culture Supernatant of HIL-3

| Concentration of culture supernatant (%) | Colonies derived from juvenile erythrocyte precursor cells | Non-erythrocyte colonies |
|---|---|---|
| 0 | 6.7 ± 1.2 | 20.7 ± 8.1 |
| 10 | 9.7 ± 2.1 | 201 ± 20 |
| 20 | 6.7 ± 0.6 | 220 ± 30 |

The data shows that the culture supernatant of HIL-3 has the ability to produce BFU-E and CFU-Mix even if it does not contain IL-2.

The IL-2 free culture supernatant of HIL-3 used in the experiment was prepared by the following method.

HIL-3 cells growing in the presence of IL-2 were washed three times with PBS and cultivated in an IL-2 free, 10% FCS-supplemented RPMI-1640 medium at a concentration of $1 \times 10^6$ cells/ml. After cultivation for 3 days, the supernatant of the culture was recovered. Throughout this cultivation period, the growth of HIL-3 cells was suspended but their viability was maintained.

It has been confirmed that the culture supernatant of HIL-3 has the ability to produce human IL-3 of high activity with high reproducibility for at least 3 days even in the absence of IL-2.

## ADVANTAGES OF THE INVENTION:

The human IL-3 having mast cell growth activity that is obtained by the present invention induces the growth and differentiation of multipotent stem cells and other hemopoietic cells in general. Besides the hemopoietic cells, this human IL-3 has the ability to stimulate the growth of other various cells. Therefore, this human IL-3 has the potential to be used not only in treating various diseases such as hypoplastic anemia, thrombocytopenia, myelocytic and lymphocytic leukemia and erythrocythemia that are caused by damage to the process of growth and differentiation of hemopoietic or other cells, but also in preventing side reactions from occurring as a result of therapeutic regimens such as chemotherapy and radiotherapy. Particularly notable results are anticipated when this human IL-3 is used for the purpose of growing marrow cells after bone marrow transplantation.

The IL-3 of the present invention are superior to the conventionally known IL-3 in that it is capable of inducing the growth of target cells and stem cells without decreasing the number of bone marrow stem cells.

The present inventors have succeeded in establishing human T cell line that has the ability to produce IL-3 which may be used to treat the various symptoms mentioned above. The present inventors have also succeeded in producing human IL-3 from this T cell line.

## Claims

1. A human cell capable of producing human interleukin 3 having mast cell growth activity, or a clone or subclone thereof.

2. A human cell according to Claim 1 which is a human leukemia T cell or a clone or subclone thereof.

3. A human cell according to Claim 1 which is human leukemia T cell line HIL-3, or a clone or subclone thereof.

4. A process for preparing human interleukin 3 having mast cell growth activity which comprises cultivating human cells capable of producing human interleukin 3 having mast cell growth activity, or a clone or subclone thereof, and recovering the resulting human interleukin 3 having mast cell growth activity.

5. A process according to Claim 4 wherein the human cells capable of producing human interleukin 3 having mast cell growth activity are interleukin 3 producing human leukemia T cells, or a clone or subclone

14

thereof.

Fig. 1

(1) (2)

0285429

(3) (4)

Fig. 3

0285429

Fig. 2

0285429

# Fig. 4

multipotent stem cells (CFU-Mix)
immature erythrocyte stem cells (BFU-E)
granulocyte-macrophage colony forming factor (CFU-GM)

Fig. 5

0285429

Concentration of STIL-β-CM (% v/v)

Fig. 6

0285429

Number of mast cell colonies/plate

Number of marrow cells (x10^4)/plate

# Fig. 7

# Fig. 8

(1)

(2)

Fig. 8

(3)

(4)

Fig. 9　　　　0285429

(1)

(2)

0285429

Fig. 9

(3)